# EUROPEAN PATENT APPLICATION

(11) **EP 3 476 408 A1**
(43) Date of publication of application: **01.05.2019**
(21) Application number: 17001754.5
(22) Date of filing: 24.10.2017
(51) Int. Cl.: A61L 27/24

(54) **A COLLAGEN FIBRIL AS WELL AS PRODUCTS, METHODS AND USES RELATING THERETO**

(71) Applicant: Universität Bielefeld, 33615 Bielefeld (DE)
(72) Inventor: Kaltschmidt, Barbara, 33619 Bielefeld (DE); Kaltschmidt, Christian, 33619 Bielefeld (DE); Hütten, Andreas, 33615 Bielefeld (DE); Greiner, Johannes, 33615 Bielefeld (DE)
(74) Representative: Teschemacher, Andrea

(57) **Abstract**

The present invention relates to a method for producing collagen type I fibrils, a collagen type I fibril, a method for producing a membrane, a membrane comprising the collagen fibril, a method for inducing osteogenic differentiation of stem cells *in vitro,* the use of the collagen fibril or of the membrane for inducing osteogenic differentiation of stem cells *in vitro* or for inducing the production of a bone *in vitro,* the collagen fibril or the membrane for use as a medicament or in a method of treatment.

## Description

The present invention relates to a method for the producing collagen type I fibrils, a collagen type I fibril, a method for producing a membrane, a membrane comprising the collagen fibril, a method for inducing osteogenic differentiation of stem cells *in vitro,* the use of the collagen fibril or of the membrane for inducing osteogenic differentiation of stem cells *in vitro* or for inducing the production of a bone *in vitro,* the collagen fibril or the membrane for use as a medicament or in a method of treatment.

Collagens such as Collagen type I are structural proteins and represent the main structural component of bone. Collagen can be found in many tissues, including cartilage, bone, cornea, muscle, and tendon and also is the principal structural component of skin. Collagen is made of triple-helix collagen molecules staggeredly assembled to fibrils leading to the occurrence of single D repeats of 67 nm (Alexander, B. et al. Journal of the Royal Society, Interface / the Royal Society 2012, 9 (73), 1774-86.). Collagen is produced by cells called osteoblasts, which are attached to bones. Besides collagen, osteoblasts additionally secrete further proteins, which together provide the natural scaffold for apatite crystals and even actively control mineralization and therefore the production of bone.

Destructions of bone tissue after accidents, infections or tumor therapy require a highly orchestrated regeneration process dependent on the presence of osteoblasts forming new bone. Since native bone harbors too few osteoblasts to enable a fast recovery of acutely injured bone tissue, stem cells need to give rise to new osteoblasts directly upon fracture or surgical treatment. In this regard, mesenchymal stem cells (MSCs) are commonly described to undergo differentiation into osteogenic cells types, such as osteoblasts (Wang, X. et al. International orthopaedics 2013, 37 (12), 2491-8.).

In addition, so called inferior turbinate stem cells (ITSCs) possess an extraordinary high differentiation potential. In particular, ITSCs are reported to efficiently undergo differentiation not only into ectodermal derivates, but also into mesodermal cell types, namely early and late osteoblasts. ITSCs belong to the broad range of craniofacial adult neural crest-derived stem cells (NCSCs) and are isolated out of the human nasal cavity. They represent a highly promising cell source for bone tissue engineering, particularly within the craniofacial region (Greiner, J. F. et al. European cells & materials 2011, 22, 403-19, Hofemeier, A. D. et al. Scientific reports 2016, 6, 26716.).

While methods for the production of membranes comprising collagen for medical purposes are known, these membranes themselves in general do not show any osteoinductive effects. For example, EP 2 703 015 A1 discloses a collagen membrane formed by drying a solution comprising completely dissolved gelatin. Therefore, the collagen used to form the membrane has due to entire dissolution completely lost its natural structure. In order to achieve stimulation of bone formation by this membrane strontium containing osteoinductive powder material is dispersed in the membrane. US 4,776,890 discloses a method for preparing a bone implant in form of a mineral-embedded collagen matrix containing the mineral component coated with collagen for hard tissue repair. This method requires collagen in its soluble form, which after mixing with mineral particles, is treated with a solution of a precipitating buffer, which raises the pH of the solution to around neutrality, in order to precipitate the collagen around the particles. Also this method works with entirely dissolved collagen, which completely looses its natural structure. Finally, US 8,834,864 B2 discloses a method of using a substantially non-porous equine collagen foil comprising collagen fibrils to repair and regenerate dura mater tissue of mammals. No use of this foil for bone regeneration and no osteoinductive effect are disclosed.

To sum up, the membranes for bone regeneration used so far are based on collagen, which is completely dissolved before it is further processed to a membrane, and consequently has completely lost its natural structure.

Further, despite the well understood structural characteristics of collagen, there was previously no direct link between a distinct nanotopography and its major role in bone recovery and growth.

The object of the present invention is to provide collagen material with a natural structure. The problem is solved by a method according to the invention, in which the collagen is essentially maintained in a solid state and not brought into solution.

In a first aspect the present invention relates to a method for producing collagen type I fibrils, the method comprising the steps of
i) providing a tendon,
ii) mechanically disrupting the tendon from step i) into pieces,
iii) enzymatically digesting the pieces of tendons from step ii) in a solution,
iv) further disrupting the enzymatically digested pieces of step iii) to produce collagen type I fibrils; and
v) collecting the collagen type I fibrils of step iv).

The data shown in the examples indicate that the method according to the invention allows the isolation of collagen type I fibrils of a certain structure. In addition, the data shown in the examples demonstrate a successful induction of osteogenic differentiation of inferior turbinate stem cells (ITSCs) when they are cultivated on the collagen fibril obtained by the method according to the invention or on the collagen fibril according to the invention.

In a first step of the method a tendon is provided. The term "tendon", as used in the context of the present invention, may be understood in the broadest sense as a connective tissue, which in general forms bands that connect muscles to bones in all parts of the body of a creature or, which commonly forms a network supporting the localization and structure of the intestine in the abdomen. The major components of a tendon are collagen fibers, whereby collagen type I fibers are the predominant collagen type. The tendon may be obtained by dissecting it from any part of a body of a creature. Preferably, the tendon may be isolated from various sources, but it is more preferably purified atelopeptide collagen, i.e. a peptide without ends, preferably from a variety of sources to reduce immunogenicity.

In preferred embodiment, the tendon of step i) is obtained from a mammal. In a more preferred embodiment, the tendon is obtained from a rat, pig, bovine, horse or human, especially human.

Preferably, the tendon is obtained from skin, gut, limb, tail, small intestine or placenta, especially from a tail, small intestine or placenta. More preferably, the tendon is obtained from fish skin, fish gut, equine limb, equine gut, pig limb, pig gut, pig tail, pig small intestine, rat tail or rat gut, especially from a rat tail, a pig tail, pig small intestine, or human placenta. Out of human sources, placenta and frozen skin might be a suitable material as well as frozen material from surgery and corpses.

While tendons from all parts of the body in general can be used to perform the method according to the first aspect of the invention, in another preferred embodiment, the tendons in step i) of the method according to the first aspect of the invention are dissected from tail, small intestine, or placenta, particularly placenta.

The dissection in general may be performed with a knife, scissor or a scalpel as known to the skilled practitioner. It may be performed in buffer. In a preferred embodiment, this buffer comprises a detergent. In a more preferred embodiment, the dissection is performed under phosphate buffer with Triton X-100, e.g. 0.1 % to 0.5 % such as 0.2 % Triton X-100.

In a second step of the method a tendon is mechanically disrupted into pieces. Any suitable method may be used for the disruption. Preferably the disrupting is performed by cutting the tendon into pieces. The maximum size of the pieces (edge length) may be as small as 3 mm, more preferably 2 mm and even more preferably 1 mm. Suitable instruments for the mechanical disruption in step ii) of the method according to the first aspect of the invention are for example knife, scissors or scalpel.

In a third step of the method the pieces of the tendon from step ii) are enzymatically digested in a solution. The term "enzymatic digestion" as used in the context of the present invention, may be understood in the broadest sense as the process of hydrolyzing macromolecules into smaller entities or their molecular components by an enzyme. The enzyme selected for the digestion will depend on the nature and source of the biological material to be digested. In the present invention a protease, a lipase and/or an amylase are usually suitable, whereas collagenase should be generally avoided. Accordingly, the enzymatically digesting of step iii) may be by incubating the tendon in solution comprising a protease and optionally a lipase and/or an amylase, preferably comprising a protease, a lipase and an amylase. Surprisingly, it has been found that the enzymes and mild concentrations thereof usually used for washing of cloths with commercially available color washing agents such as Persil Megaperls® (Henkel, Germany) are particularly suitable in this context. These enzymes evidently allow for higher incubation temperatures. A suitable amount of washing agent for enzymatic digestion is 1-5 Megaperls, more preferred 2-3, most preferred 2 Megaperls were dissolved in 1ml buffer.

The term "protease" as used in the context of the present invention, may be understood in the broadest sense as an enzyme catalyzing the hydrolysis of a peptide bond within a protein or peptide leading to peptides or single amino acids. In general, this reaction may be unspecific, i.e. that any peptide bond within the protein or peptide may be hydrolyzed. The protease may be a protease of the subtilase family, more preferably an alkaline protease of a Bacillus species.

Depending on their catalytic residue, proteases can be classified into seven protease classes, such as serine proteases, which use the alcohol residue of serine to catalyze the hydrolysis reaction. Subtilases belong to the family of subtilisin-like serine proteases of the class of serine proteases and generally are characterized by a catalytic triad of the amino acids aspartate, histidine and serine. Their pH optimum in general is in the alkaline pH range. Originally, subtilases are produced and secreted by microorganisms, such as bacteria from the Bacillus species.

The term "lipase" as used in the context of the present invention, may be understood in the broadest sense as an enzyme catalyzing the hydrolysis of the ester bond between a triglyceride and a fatty acid.

The term "amylase" as used in the context of the present invention, may be understood in the broadest sense as an enzyme catalyzing the hydrolysis of polysaccharides.

Preferably, the buffer used in step iii) of the method according to the first aspect of the invention does not comprise any collagenase. The term "collagenase" as used in the context of the present invention, may be understood in the broadest sense as an enzyme catalyzing the peptide bond connecting the amino acid proline with an adjacent amino acid. Collagenases are especially suitable for the enzymatic digestion of collagen due to its high proline content.

Depending on its amino acid composition and structure, each enzyme has a different and characteristic pH optimum for its enzymatic function. If the pH value of the environment of the enzyme only slightly deviates from this pH optimum, the speed of the reaction catalyzed by the enzyme may significantly decrease. Therefore, enzymatic reactions can be performed in buffer in order to maintain a pH, which is optimal for the enzyme used in the reaction. In a preferred embodiment, the enzymatic digestion in step iii) is an incubation of tendons in solution comprising a buffer, such as a borate buffer or a 2-amino-2-(hydroxymethyl)propane-1,3-diol (TRIS)/HCl buffer, which even more preferred TRIS (such as 30 mM to 100mM, especially about 50 mM) and a pH of 8 to 9, especially preferred 8.5.

In a preferred embodiment, the solution in step iii) comprises an ionic surfactant and/or a non-ionic surfactant. The term "surfactant" as used in the context of the present invention, may be understood in the broadest sense as a surface active agent, i.e. an agent decreasing the surface tension or interfacial tension between two phases, which might be both liquids or one liquid and one solid. This allows e.g. dispersion or dissolving of both phases. In general, surfactants comprise a head portion, which interacts with one phase, and a tail portion, which interacts with the other phase. The term "non-ionic surfactant" as used in the context of the present invention, may be understood in the broadest sense as a surface active agent, which does not have an electrical charge in its head portion. In contrast, the term "ionic surfactant" as used in the context of the present invention, may be understood in the broadest sense as a surface active agent, which has an electrical charge in its head portion. Non-ionic surfactants suitable for use in a buffer in step iii) of the method according to the first aspect of the invention are alkylphenol ethoxylates, such as Triton X-100.

In another preferred embodiment, the solution used in step iii) of the method according to the first aspect of the invention comprises a protease, a lipase, an amylase, a ionic surfactant and/or a non-ionic surfactant.

The reaction conditions, such as temperature and reaction time, applied for the enzymatic digestion in step iii) of the method according to the first aspect of the invention among others depend on the enzymes used for enzymatic digestion. In general, the reaction time is from 10 min to 12 hours, more preferably from 30 to 180 minutes, particularly 90 to 210 min such as about 2 hours. In general, the reaction may be performed at 15 to 60°C, more preferably at room temperature (25°C) to body temperature (e.g. 37°C) and especially preferred at 37°C.

In another preferred embodiment, the incubation in step iii) of the method according to the first aspect of the invention is performed for about 2 hours at about 37°C.

Reaction parameters for the enzymatic digestion in step iii) of the method according to the first aspect of the invention, such as buffers, enzymes, pH, reaction temperature and reaction time are well known by the person skilled in the art as well as their combination dependent from the enzymes used.

The enzymatic digestion in step iii) of the method according to the first aspect of the invention may be followed by a step comprising washing of the material obtained in step iii) of the method according to the first aspect of the invention.

Suitable washing buffer is the buffer used for enzymatic digestion in step iii), but without enzymes. Preferably, a 50 mM Tris buffer having a pH of 8 to 9, more preferably a pH of 8.5, adjusted with HCl is used. Preferably, the material obtained in step iii) of the method according to the first aspect of the invention is washed in 3 to 5 washing steps.

In another preferred embodiment the enzymatic digestion of step iii) of the method according to the first aspect of the invention is followed by a step comprising washing of the material obtained in step iii) (i.e. the enzymatically digested pieces of tender) of the method according to the first aspect of the invention in at least 1 (e.g. 3, 4 or 5) washing steps. The washing may be done with a solution comprising Tris buffer (e.g. 50 mM; pH of 8 to 9).

In a fourth step of the method the enzymatically digested pieces of step iii) are further disrupted to produce collagen type I fibrils. The further disruption may be performed with any mechanical method. Suitable methods for the disruption are known to the person skilled in the art.

Examples for suitable mechanical methods are grinding of the material obtained in step iii), e.g. in a mortar in a first step, whereby, preferably, the material is cooled (e.g. with liquid nitrogen). This first step may optionally be followed by a second step comprising breaking down of the grinded material of the first step into fibrils. Suitable for this second step are techniques based on ultrasonic disintegration. Preferably an ultrasonic bath or a probe type ultrasonic device may be used. Suitable peak to peak amplitudes are known by the person skilled in art. Preferably, the peak to peak amplitude is from 20 to 40 microns, more preferably from 25 to 35 microns and especially preferred 30 microns.

Buffers suitable for use for further disruption of the material obtained in step iii) are phosphate buffers. Preferably, a phosphate buffer with Triton X-100 (e.g. 0.2 %) is used.

Preferably, the further disrupting of step iv) comprises grinding of the enzymatically digested pieces of step iii), and optionally breaking down of the grinded disrupted tendons into fibrils. The breaking down of specimens into fibrils is performed by ultrasonic disintegration in an ultrasonic bath or a probe type ultrasonic device, preferably at peak to peak amplitude from 20 to 40 microns, more preferably from 25 to 35 microns and especially preferred 30 microns and/or preferably in phosphate buffer with 0.2 % Triton X-100 for 30 seconds at 4° C.

In a fifth step of the method, the collagen type I fibrils of step iv) are collected. The collecting may preferably be done by centrifugation. Methods for concentrating collagen solutions are well known to the person skilled in the art. Preferably, the centrifugation in step v) is performed at 5000 g to 20000 g, preferably at 8000 g to 17000 g, more preferably at 10000 g to 15000 g most preferably at 12000 g to 14000 g and especially preferred at 13000 g.

The collagen fibrils produced according to the first aspect of the invention may be further concentrated e.g. by lyophilization. Suitable methods for lyophilisation are known to the person skilled in the art. Preferably, surplus water is sucked away allowing a very thin film of remaining water on the sample. The specimen may be immediately plunge-frozen e.g. in liquefied propane, more preferably at -190° Celsius. In general, the shock frozen specimen is transferred under N₂-atmosphere into a freeze-drying apparatus, which preferably has a temperature of -196° Celsius. The device may then be brought into a high-vacuum chamber and drying ("sublimation") may take place over 15 hours at 10⁻⁵ millibar. Exemplary and suitable devices for lyophilisation are available from Martin Christ Gefriertrocknungsanlagen GmbH (Osterode am Harz, Germany).

In a second aspect the present invention relates to a collagen fibril obtainable by a method according to the first aspect of the invention.

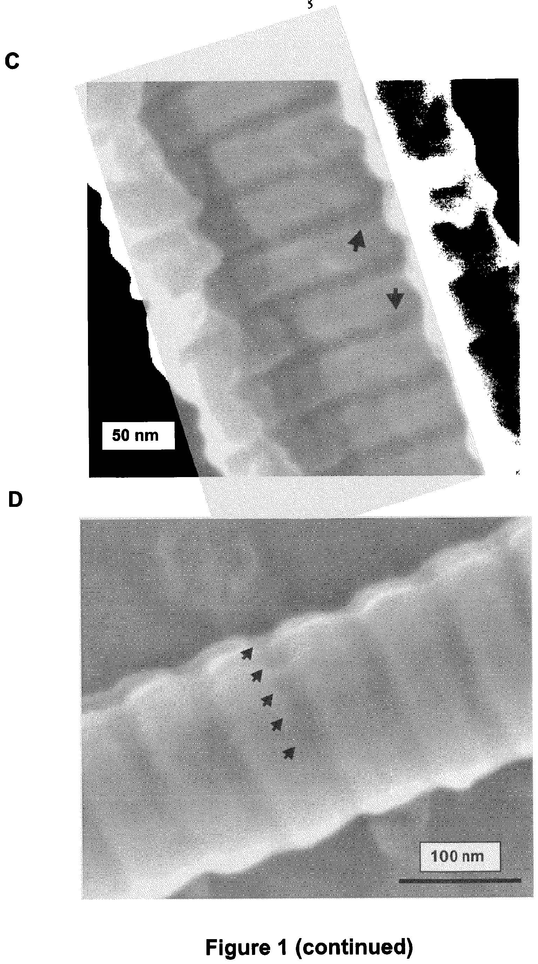

In a third aspect, the invention relates to an isolated collagen fibril characterized in that it is a collagen type I fibril, which is cross-striated and which has pores having a diameter of 25 to 35 nm, preferably of 27 to 33 nm, more preferably of 28 to 32 nm, most preferably of 29 to 31 nm and especially preferred of 30 nm. The pores are shown in Figures 1C and 1D (see arrowheads). In accordance with the present invention, the collagen fibril does preferably not comprise strontium.

In a fourth aspect, the invention relates to a method for producing a membrane, the method comprising the steps of
i) forming a gel from the collagen fibril according to the second aspect of the present invention,
ii) converting the gel of step i) to the membrane, preferably by compressing the gel of step i) at a pressure of 1.1 to 3 atmospheres until the volume is 1 to 10%, preferably 3 to 7%, more preferably 4 to 6% and especially preferred 5% of the gel of step i),
iii) optionally stabilizing the membrane by crosslinking; and
iv) drying the compressed gel.

In a first step a gel is formed. Methods for the formation of a gel from a collagen solution in step i) of the method according to the fourth aspect of the invention are known to the person skilled in the art. Suitable methods are described in US 4,655,980.

In general, gel formation may be obtained by three alternative methods. First, the pH of the collagen solution may be raised by treatment with a precipitating buffer, leading to the insolubility of the collagen. At the moment of mixing, collagen solution and buffer may be pre-cooled almost to freezing, whereby incubation for gel formation may take place at room temperature (e.g. 18 to 26 °C). Second, the collagen solution may be centrifuged. Afterwards, the supernatant may be isolated and incubated at room temperature. Third, the collagen solution is brought to physiological pH and ionic strength by treatment with an insolubilizing solution at room temperature. Gel formation is achieved by incubation e.g. at 37°C. The third method may be modified by degassing the mixture immediately upon mixing. Further, the degassed mixture may be placed into a mold before incubation.

Preferably, gel formation according to step i) of the method according to the fourth aspect of the invention is performed at temperatures from 4°C to 37°C.

Gel formation is then followed by conversion of the gel to a membrane. Methods for the conversion of the gel to a membrane in step ii) of the method according to the fourth aspect of the invention are known to the person skilled in the art or described in US 4,655,980.

Conversion of the gel to a membrane may be accomplished by two basic alternative methods. First, compression of the gel under constant pressure and followed by drying leads to a gel mat. A solid implant may be achieved instead by compressing the molded gel obtained from the modification of the gel formation process which employs degassing. A fiber product is obtained, if the pressure is applied around the circumference of a cylinder formed from the gel. The second method of converting the gel into a membrane comprises disruption of the gel followed by centrifugation leading to a precipitate, which may be casted into molds and dried. Depending on the dimensions and shape of the mold, either a membrane or solid can be obtained.

Preferably, the conversion of the gel to a membrane in step ii) of the method according to the fourth aspect of the invention is performed by the first alternative, i.e. compression of the gel under constant pressure and followed by drying. For compression, the gel may be squeezed in a suitable piston device, such as an apparatus used to obtain tofu. Preferably, compression is conducted using 1.1 to 3 atmospheres pressure. In general, the conversion of the gel to a membrane is continued until the volume of the membrane is 1% to 10%, preferably 3 to 7%, more preferably 4 to 6% and especially preferred 5% of the gel obtained in step i) of the method according to the fourth aspect of the invention.

Alternatively, plastification can be also done by reducing volume with a filter paper with a weight on top, preferably by filling collagen in solution/suspension in a tissue plate putting a nylon screen on top adding a filter and placing a weight on top.

As a third step, the collagen in the membrane may be optionally stabilized by crosslinking, e.g. with glutaraldehyde. Preferably, the membrane is treated with a solution containing 0.05 to 1% glutaraldehyde for 1 to 16 hours, and then quenched by addition of a glycine solution to a concentration of about 0.1 to 0.4 M glycine. Preferably, the cross-linking solution is then removed by washing.

In the fourth step of the method according to the fourth aspect of the invention the resulting membrane from step ii) or iii) of the method according to the fourth aspect of the invention may then be dried, preferably in air and more preferably at a temperature below 37°C. Optionally, step iv) of the method according to the fourth aspect of the invention may be followed by a washing step, comprising washing of the dried membrane, e.g. with water, to remove salts and other contaminants, followed by redrying, such as in air, at low temperature.

In a fifth aspect, the invention relates to a membrane obtainable by the method for the production of a membrane according to the fourth aspect of the invention.

In a sixth aspect, the invention relates to a membrane comprising the collagen fibril according to the second aspect or the collagen fibril according to the third aspect of the invention.

In another preferred embodiment, the membrane according the fifth aspect or the membrane according to the sixth aspect of the invention is cell-free and/or does not comprise strontium and/or is incrusted with calcium apatite microcrystals.

Hydroxylapatite is a bone mineral and a major component of a bone. It may be used as filler for bone lesions. Further, implants may generally be covered with hydroxylapatite as this may promote bone regeneration.

In a seventh aspect, the invention relates to a method for inducing osteogenic differentiation of stem cells *in vitro,* the method comprising the steps of
i) seeding the stem cells on the collagen fibril according to the second or the third aspect of the invention or on the membrane according to the fifth or sixth aspect of the invention, and
ii) cultivating the seeded cells.

The term "osteogenic differentiation" as used in the context of the present invention, may be understood in the broadest sense as the differentiation of a stem cell into any cell involved in bone growth or bone repair. Suitable stem cells are for example mesenchymal stem cells (MSCs) or ITSCs, preferably from human or murine origin.

Methods for the cultivation of stem cells are well known to the person skilled in the art. This also includes various parameters, such as medium, temperature, atmosphere or duration of cultivation until the desired effect is achieved.

Preferably, the cultivation of stem cells in step ii) of the method according to the seventh aspect of the invention is performed at 37 °C and 5% CO₂ in a humidified incubator.

In one embodiment the cells in step i) of the method according to the seventh aspect of the invention are seeded without osteogenic differentiation medium and by pure physical cues.

In another preferred embodiment, the seeded cells in step ii) of the method according to the seventh aspect of the invention are cultivated for at least 7 days, preferably at most 28 days, e.g. in Dulbecco's Modified Eagle's Medium with 10% fetal calf serum.

Preferable methods for the detection of the induction of osteogenic differentiation are the detection of alkaline phosphatase (ALP) activity after seven days of cultivation or Alizarin red S staining of calcium deposits by applying fast red violet lb staining solution after 21 days of cultivation.

Preferably, the method according to the seventh aspect of the invention is for the *in vitro* production of bones.

In an eighth aspect, the invention relates to the use of the collagen fibril according to the second aspect of invention, of the collagen fibril according to the third aspect of the invention, of the membrane according to the fifth aspect of invention or of the membrane according to sixth aspect of the invention for inducing osteogenic differentiation of stem cells *in vitro* or for inducing the production of a bone *in vitro.*

In another preferred embodiment the method according to the seventh aspect of the invention or the use according to the eighth aspect of the invention the stem cells are inferior turbinate stem cells (ITSCs) and/or mesenchymal stem cells (MSCs), preferably from human or murine origin.

In another preferred embodiment the method according to the seventh aspect of the invention or the use according to the eighth aspect of the invention at least one cellular signaling pathway is activated during the inducing, preferably wherein Fak1 is phosphorylated and/or the expression of the gene Osterix is increased.

The term "cellular signaling pathway" as used in the context of the present invention, may be understood in the broadest sense as a communication process eliciting a cell response to an extracellular signal. In general, signaling pathways start with the detection of an extracellular signal by a receptor that forwards the signal to a cascade of various proteins, which direct the signal into the cell in order to elicit the appropriate cellular response.

The protein focal adhesion kinase 1 (Fak1) is a protein kinase involved in signaling pathways regulating cell adhesion and cell motility processes. Fak1 is an intracellular protein, mainly located in focal adhesions, which are sub-cellular structures linking a cell to the extracellular matrix. In addition, Fak1 plays a major role in signaling pathways directing differentiation and proliferation of many cell types, such as osteoblast progenitor cells.

Post-translational modifications of proteins, such as the phosphorylation of an amino acid, control protein activity, location or its interaction with other proteins. In general, a protein may be phosphorylated at several positions of its amino acid sequence, whereby each phosphorylation may have a different effect. In case of Fak1, many phosphorylation sites are known, which control various specific processes, such as enzymatic activity or degradation, or even more general processes, such as cell differentiation.

Methods for the detection of the phosphorylation of proteins are well known to the person skilled in the art. For example, these comprise mass spectrometry, but also further techniques, such as Western Blot, ELISA or flow cytometry, e.g. with antibodies specifically targeting the phosphorylated site.

Osterix is a protein, which generally acts as a transcription factor. For example, it is known to induce the differentiation of mesenchymal precursor cells into osteoblasts. The genes Osterix and alkaline phosphatase are hallmarks of osteogenic differentiation and are characterized by the presence of kappaB sequences binding the transcription factor NF-kappaB, most preferably NF-kappaB c-REL.

Methods for the detection of gene expression are well known to the person skilled in the art. For example, these comprise Northern Blotting, RT-qPCR or determining the localization of a protein by In situ hybridization or immunofluorescence techniques.

In a ninth aspect, the invention relates to a collagen fibril according to the second aspect of the invention, a collagen fibril according to the third aspect of the invention, a membrane according to the fifth aspect of the invention or a membrane according to the sixth aspect of the invention for use as a medicament.

In a tenth aspect, the invention relates to a collagen fibril according to the second aspect of the invention, a collagen fibril according to the third aspect of the invention, a membrane according to the fifth aspect of the invention or a membrane according to the sixth aspect of the invention for use
- in a method for inducing osteogenic differentiation of stem cells,
- in a method for the production of a bone,
- in a method of treating an accidental injury, preferably of treating a bone fracture or for bone replacement,
- in a method of replacing a bone tissue, preferably after infection or during or after tumor therapy,
- in a method involving joint replacement,
- in a method involving a dental or hip prosthesis, and/or
- in a method involving plastic surgery in the head region.

Preferably, the membrane according to the fifth or sixth aspects of the invention is biodegradable and / or resorbable and, more preferably, usable for guided tissue regeneration and/or prevention of adhesions, such as adhesion of soft tissue.

Suitable are also membranes according to the fifth or sixth aspect of the invention, which have two different sides. For skull injuries, preferably a hybrid membrane can be constructed, which may comprise structured collagen to induce bone formation on one side and unstructured collagen on the other side in order to avoid bone formation enabling soft tissue maintenance. This may allow to isolate brain tissue with a non bone formig membrane on top of that the bone forming collagen is deposited. Structured collagen as used in the context of membranes having two different sides, may be understood in the broadest sense as collagen comprising nanopores. Unstructured collagen as used in the context of membranes having two different sides, may be understood in the broadest sense as collagen, which is soluble and does not comprise nanopores. Further suitable is a collagen membrane with one side for primary liquid tightness preventing cerebrospinal fluid leakage and adhesion formation and the other side inducing bone formation.

An osteoinductive collagen membrane for haemostasis and as a carrier for fibrin sealant may be suitable for various forms of surgery as well.

Further, the membrane according to the fifth or sixth aspect of the invention may be combined with biological agents, such as bone morphogenetic proteins suitable for bone regeneration. Suitable agents are well known to the person skilled in the art.

The invention is not limited to the particular methodology, protocols and reagents described herein because they may vary. Furthermore, the terminology used herein is for the purpose of describing particular embodiments only and is not intended to limit the scope of the present invention. As used herein and in the appended claims, the singular forms "a", "an", and "the" include plural reference unless the context clearly dictates otherwise. Similarly, the words "comprise", "contain" and "encompass" are to be interpreted inclusively rather than exclusively.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, and materials are described herein.

The present invention is further illustrated by the following Figures and Examples, from which further features, embodiments and advantages may be taken. As such, the specific modifications discussed are not to be construed as limitations on the scope of the invention. It will be apparent to the person skilled in the art that various equivalents, changes, and modifications may be made without departing from the scope of the invention, and it is thus to be understood that such equivalent embodiments are to be included herein.

### FIGURES

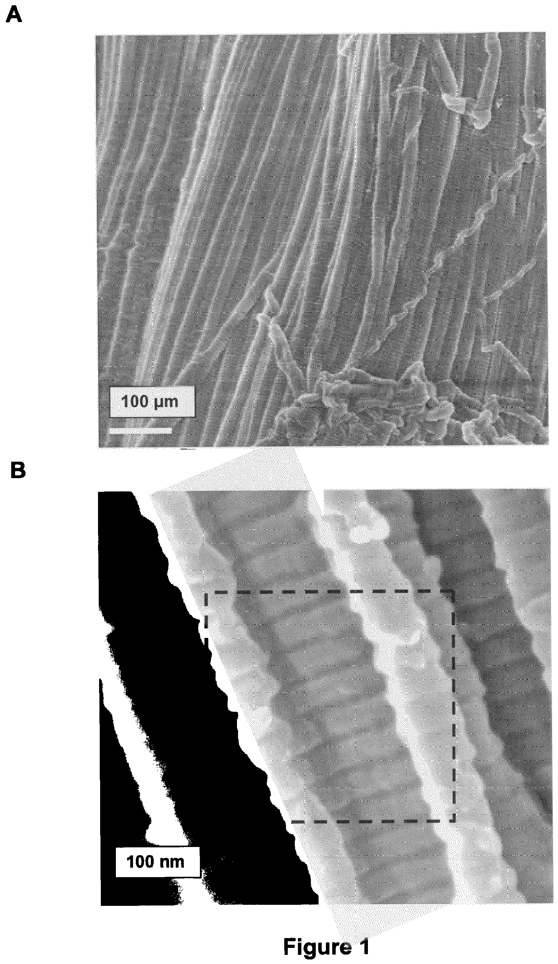

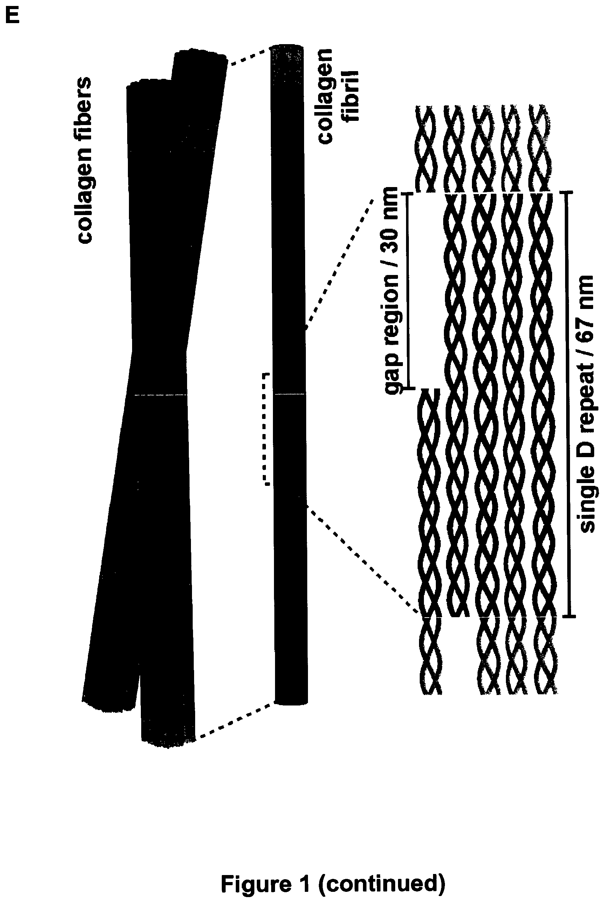

**Figure 1****:** Collagen fibrils show pore-like structures of approximately 30 nm size. A-B: Collagen type I fibers and fibrils imaged by scanning electron microscopy (SEM). C-D: Pore-like structures of approximately 30-33 nm (arrows) were detectable within the cross-striated pattern of collagen fibrils. Please note that Fig. 1C corresponds to the marked portion of Fig 1B. E: Scheme of two collagen fibers (top), which comprise collagen fibrils (middle), which further are made of triple-helix collagen molecules. The staggered assembly of the triple-helix collagen molecules leads to the occurrence of single D repeats of 67 nm as well as 30 nm gap regions (bottom).

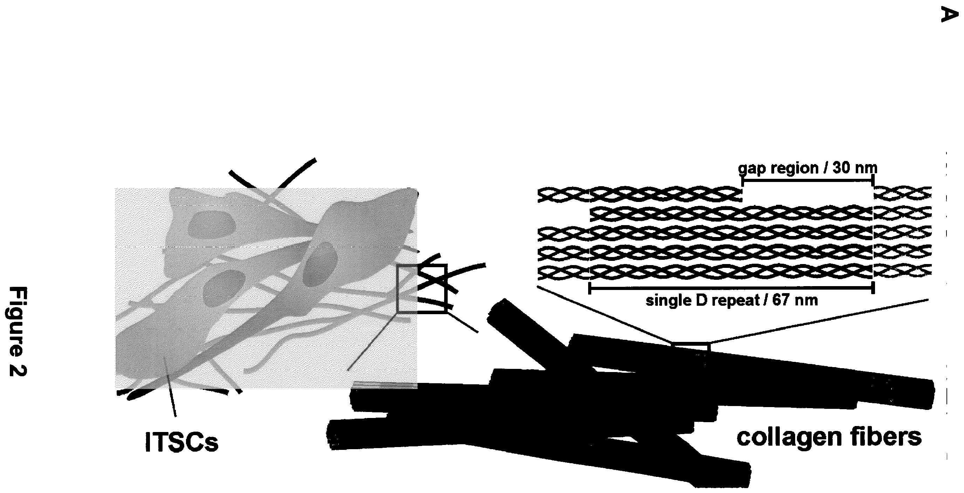

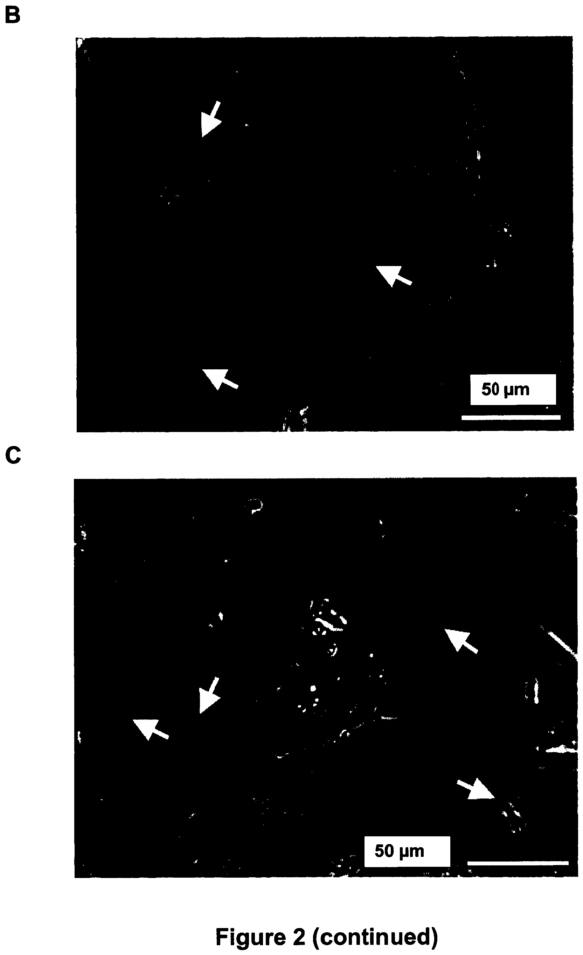

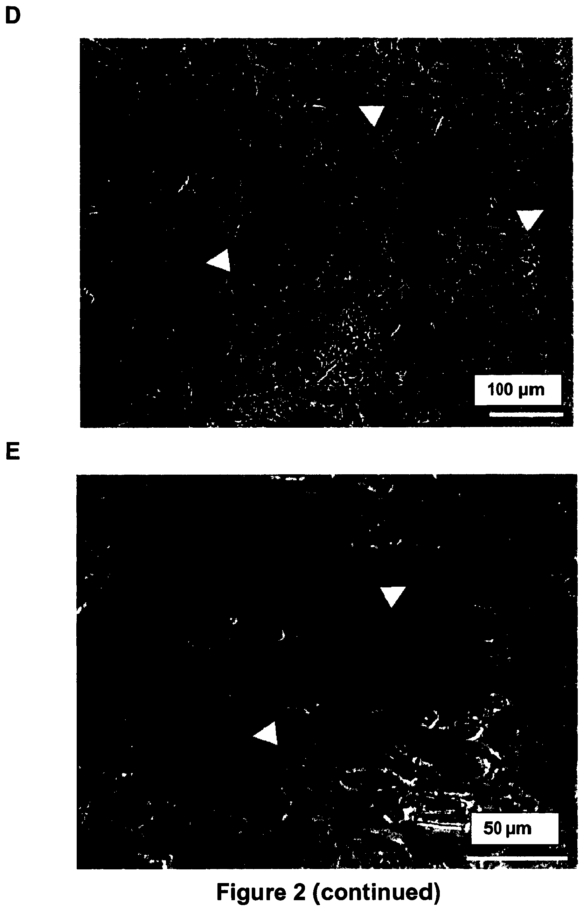

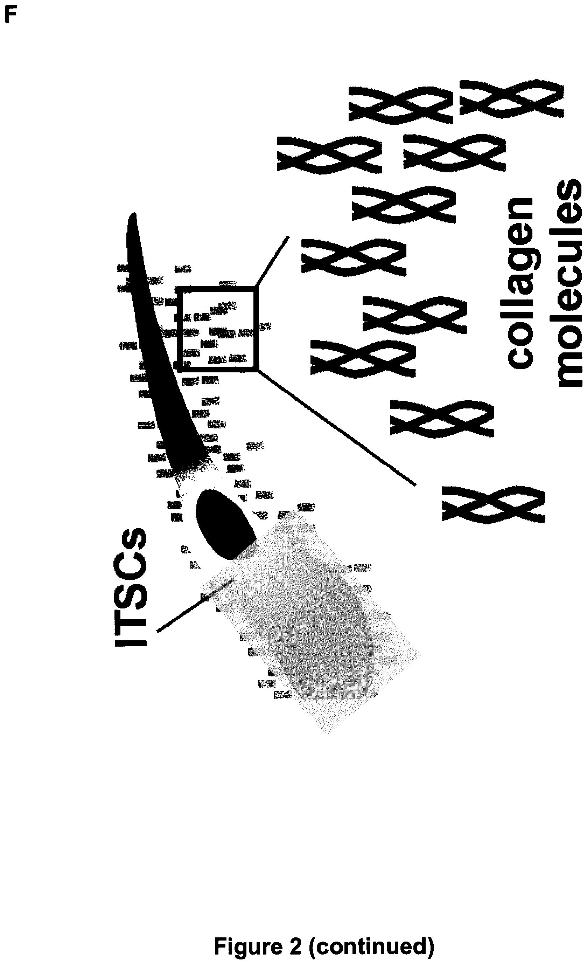

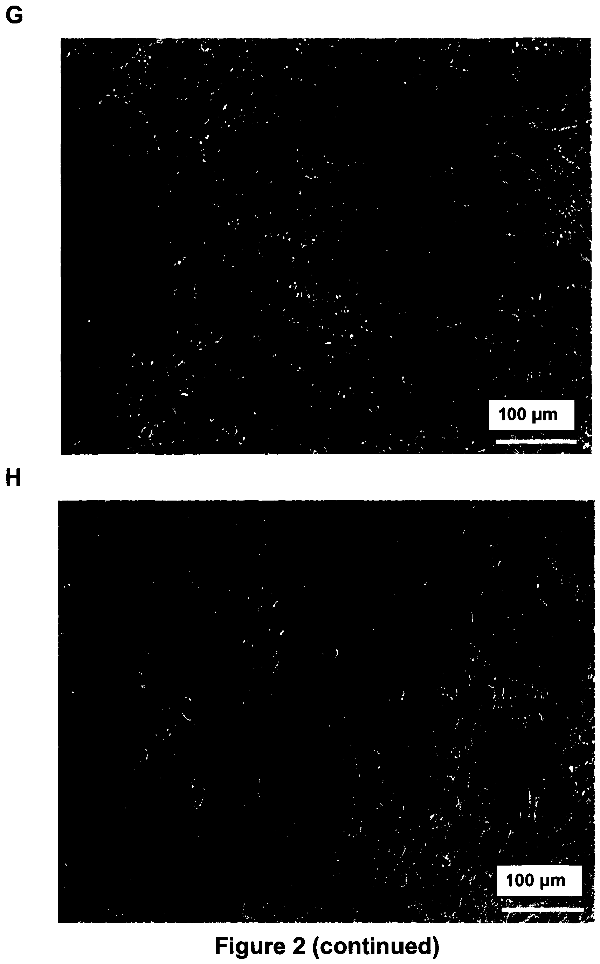

**Figure 2****:** Cultivation of human ITSCs on collagen fibrils obtained by the method according to the invention induces osteogenic differentiation.
   A: Left: Scheme of cultivation of ITSCs (cell) on collagen fibrils (lines in direct contact with ITSCs). Right: Scheme of collagen fibers comprising collagen fibrils, which are further made of triple-helix collagen molecules.
   B: Microscopic image of collagen fibrils (arrow) obtained as described in example 1.
   C: Microscopic image of ITSCs cultivated on collagen fibrils (arrow) obtained as described in example 1.
   D and E: Microscopic image of ITSCs cultivated on collagen fibrils obtained as described in example 1 and stained with Alizarin Red S as described in example 6. Alizarin Red S-positive calcium deposits (arrowheads) were observable after 21 days of cultivation of ITSCs on collagen fibrils obtained as described in example 1, demonstrating a successful induction of osteogenic differentiation.
   F: Scheme of cultivation of ITSCs (cell) on a non-collagenous surface or sole collagen molecules lacking a defined nanotopography (represented by triple helices).
   G and H: Microscopic image of ITSCs cultivated on a non-collagenous surface or sole collagen molecules lacking a defined nanotopography and stained with Alizarin Red S. No Alizarin Red S-positive calcium deposits were observable after 21 days of cultivation of ITSCs on collagen molecules lacking a defined nanotopography.

### EXAMPLES

### Example 1: Production of pure collagen fibrils

Rat tail tendons were dissected under phosphate buffer with 0.2 % Triton X-100 and care was taken to keep specimens in a wet state. Tendons were cut into pieces and incubated for 2 hours at 37° Celsius in digestion solution (borate or Tris / HCl buffer of preferably 50 mM) comprising a protease, a lipase and an amylase but no collagenase. After thorough washing specimens were grinded in a liquid nitrogen cooled mortar and further broken down into fibrils in a MSE ultrasonic disintegrator in phosphate buffer with 0.2 % Triton X-100 for 30 seconds at 4° C. Pure collagen fibrils were collected by centrifugation.

### Example 2: Concentration of pure collagen fibrils

In order to concentrate the pure collagen fibrils of Example 1, these were sedimented in distilled water onto plasma cleaned cover slides. Surplus water was sucked away from the edge allowing a very thin film of remaining water and the specimen was immediately plunge frozen in liquefied propane at -190° Celsius. The shock frozen specimen was transferred under N₂-atmosphere into a self-made freeze-drying apparatus at -196° Celsius. The device was brought into a high-vacuum chamber and drying ("sublimation") took place over 15 hours at 10⁻⁵ mbar. For cell culture experiments detergent treatment and freeze substitution were omitted.

### Example 3: Examination of pure collagen fibrils

To examine microstructurally the pure collagen fibrils resulting from example 1, these were imaged employing a scanning electron microscope (SEM, FEI Helios Nanolab 600, FEI, Hillsboro, Oregon, USA) in secondary electron mode. The pore size distribution was determined by measuring the diameter of single pores in randomly chosen ROIs with a size of 1 - 4 µm². The total count number per membrane type was chosen to be at least 150. 2 nm of Tantalum were sputtered on the collagen fibrils to avoid charging effects in the SEM. The results of scanning electron microscopy detection are shown in Figure 1A to D.

### Example 4: Isolation and cultivation of neural crest-derived human ITSCs

Human inferior turbinates were obtained during routine surgery after an informed consent according to local and international guidelines (Bezirksregierung Detmold/Münster). Isolation of ITSCs and further experimental procedures were ethically approved by the ethics commission of the Ärztekammer Westfalen-Lippe and the medical faculty of the Westfälische Wilhems-Universität (approval reference number 2012-15-fS). Isolation procedures and initial precultivation were performed as described in Hauser et al. (Hauser, S. et al. Stem cells and development 2012, 21 (5), 742-56.). Isolated ITSCs were expanded within a 3D human blood plasma-derived fibrin matrix according to Greineret al. (Greiner, J. F. et al. European cells & materials 2011, 22, 403-19).

### Example 5: Osteogenic differentiation

To topologically induce osteogenic differentiation using collagen fibrils, ITSCs were seeded onto rat collagen fibrils prepared as described in example 1 or onto standard acetic-acid dissolved rat tail collagen (sole collagen molecules) coated on cover slides and cultivated in DMEM with 10% fetal calf serum 37 °C and 5% CO₂ in a humidified incubator (Binder, Tuttlingen, Germany).

### Example 6: Detection calcium deposition by Alizarin red S staining

After 21 days of osteogenic differentiation according to example 5, ITSCs were treated with 4% paraformaldehyde for 15 min followed by washing using ddH₂O and application of Alizarin Red staining solution (1% Alizarin Red in ddH2O, pH 4.3) for 5 min at room temperature. Images were obtained using AMG EVOS xl microscope (PeqLab Biotechnology) and further processed utilizing Image J 37 with the plugin "Chart_White_Balance". Results of experiments investigating ITSCs cultivation on collagene obtainable by the method according to the invention are shown in Figure 2.

## Claims

1. A method for producing collagen type I fibrils, the method comprising the steps of
i) providing a tendon,
ii) mechanically disrupting the tendon from step i) into pieces,
iii) enzymatically digesting the pieces of tendons from step ii) in a solution,
iv) further disrupting the enzymatically digested pieces of step iii) to produce collagen type I fibrils; and
v) collecting the collagen type I fibrils of step iv).

2. The method according to claim 1,
a) wherein the tendon of step i) is obtained from a mammal, preferably a rat, pig, bovine, horse or human, especially human,
b) wherein the tendon of step i) is obtained from skin, gut, limb, tail, small intestine or placenta, especially from a tail, small intestine or placenta, more preferably from a rat tail, a pig tail, from pig small intestine, or human placenta;
c) wherein the mechanical disrupting in step ii) is performed by cutting tendons into pieces having a maximum size (edge length) of 3 mm, more preferably 2 mm and even more preferably 1 mm;
d) wherein the enzymatic digesting of step iii) is by incubating the tendon in solution comprising a protease and optionally a lipase and/or a amylase, preferably comprising a protease, a lipase and an amylase and/or preferably the protease being a protease of the subtilase family, more preferably an alkaline protease of a Bacillus species;
e) wherein the solution in step iii) comprises a borate or a 2-amino-2-(hydroxymethyl)propane-1,3-diol (TRIS) /HCl buffer, more preferably TRIS and a pH of 8 to 9, especially preferred a pH of 8.5,
f) wherein the incubation in step iii) is performed at room temperature or body temperature for a time of from 30 to 180 minutes, particularly 90 to 210 min such as about 2 hours at 37°;
g) wherein the solution in step iii) comprises an ionic surfactant and/or a non-ionic surfactant;
h) wherein the enzymatic digesting of step iii) is followed by a step comprising washing the enzymatically digested pieces of step iii) in at least 1 washing step, preferably in a solution comprising a Tris buffer; and/or
i) whereby the further disrupting of step iv) comprises grinding of the enzymatically digested pieces of step iii), and optionally breaking down of the grinded disrupted tendons into fibrils.

3. A collagen fibril obtainable by a method according to claim 1 or 2.

4. An isolated collagen fibril **characterized in that** it is a collagen type I fibril, which is cross-striated and which has pores having a diameter of 25 to 35 nm, preferably of 27 to 33 nm, more preferably of 28 to 32 nm, most preferably of 29 to 31 nm and especially preferred of 30 nm.

5. The collagen fibril according to claim 3 or 4 **characterized in that** it does not comprise strontium.

6. A method for producing a membrane, the method comprising the steps of
i) forming a gel from the collagen fibril according to any of claims 3 to 5,
ii) converting the gel of step i) to the membrane, preferably by compressing the gel of step i) at a pressure of 1.1 to 3 atmospheres until the volume is 1 to 10%, preferably 3 to 7%, more preferably 4 to 6% and especially preferred 5% of the gel of step i),
iii) optionally stabilizing the membrane by crosslinking, and
iv) drying the compressed gel.

7. A membrane obtainable by the method according to claim 6.

8. A membrane comprising the collagen fibril according to any of claims 3 to 5.

9. The membrane according to claim 7 or 8,
a) wherein it is cell-free,
b) wherein it does not comprise strontium, and/or
c) wherein it is incrusted with calcium apatite microcrystals.

10. A method for inducing osteogenic differentiation of stem cells *in vitro,* the method comprising the steps of
i) seeding the stem cells on the collagen fibrils according to any of claims 3 to 5 or on the membrane according to any one of claims 7 to 9, and
ii) cultivating the seeded cells.

11. The method according to claim 10,
a) wherein the seeded cells are cultivated for at least 7 days, preferably at most 28 days; and/or
b) wherein the method is for the *in vitro* production of bones.

12. Use of the collagen fibril according to any of claims 3 to 5 or of the membrane according to any one of claims 7 to 9 for inducing osteogenic differentiation of stem cells *in vitro* or for inducing the production of a bone *in vitro.*

13. The method according to claim 10 or 11 or the use according to claim 12
a) wherein the stem cells are inferior turbinate stem cells (ITSCs) and/or mesenchymal stem cells (MSCs), preferably from human or murine origin; and/or
b) wherein during the inducting at least one cellular signaling pathway is activated, preferably wherein Fak1 is phosphorylated and/or the expression of the gene Osterix is increased.

14. A collagen fibril according to any one of claims 3 to 5 or the membrane according to any one of claims 7 to 9 for use as a medicament.

15. A collagen fibril according to any one of claims 3 to 5 or the membrane according to any one of claims 7 to 9 for use
- in a method for inducing osteogenic differentiation of stem cells,
- in a method for the production of a bone,
- in a method of treating an accidental injury, preferably of treating a bone fracture or for bone replacement,
- in a method of replacing a bone tissue, preferably after infection or during or after tumor therapy,
- in a method involving joint replacement,
- in a method involving a dental or hip prosthesis, and/or
- in a method involving plastic surgery in the head region.
